# FASCICULE DE BREVET EUROPEEN

(11) **EP 1 513 932 B1**
(45) Date de publication et mention de la délivrance du brevet: **08.07.2015**
(21) Numéro de dépôt: 03756011.7
(22) Date de dépôt: 27.05.2003
(51) Int. Cl.: C12N 15/00, A01K 67/027, C12N 15/85, G01N 33/50, C12N 5/10

(54) **EMBRYONS TRANSGÉNIQUES DE XÉNOPE ET LEURS UTILISATIONS POUR LA DÉTECTION DE PERTURBATEURS ENDOCRINIENS DANS L'ENVIRONNEMENT**
TRANSGENE XENOPUS EMBRYOS ZUR ERKENNUNG VON ENDOKRINEN UMWELTSTÖRUNGEN
TRANSGENIC CLAWED FROG EMBRYOS AND USE THEREOF AS DETECTORS OF ENDOCRINE DISRUPTERS IN THE ENVIRONMENT

(30) Priorité: 30.05.2002 FR 0206669
(43) Date de publication de la demande: 16.03.2005
(73) Titulaire: CENTRE NATIONAL DE LA RECHERCHE SCIENTIFIQUE -CNRS-, 75016 Paris (FR); MUSEUM NATIONAL D'HISTOIRE NATURELLE, 75231 Paris Cedex 05 (FR)
(72) Inventeur: DEMENEIX, Barbara, F-75013 Paris (FR); TURQUE, Nathalie, F-75007 Paris (FR)
(74) Mandataire: Regimbeau
(86) Numéro de dépôt international: PCT/FR2003/001598
(87) Numéro de publication internationale: WO 2003/102176

(56) Documents cités:
- EP-A- 1 130 086
- EP-A- 1 180 684
- WO-A-01/88115
- WO-A-93/03179
- WO-A-98/28971
- WO-A-98/30715
- US-A- 5 094 944
- US-A- 5 571 722
- US-A- 5 612 184
- OOFUSA K, TOOI O, KASHIWAGI A, KASHIWAGI K, KONDO Y, WATANABE Y, SAWADA T, FUJIKAWA K, YOSHIZATO K.: "Expression of thyroid hormone receptor betaA gene assayed by transgenic Xenopus laevis carrying its promoter sequences." MOL CELL ENDOCRINOL, vol. 181, no. 1-2, 5 juillet 2001 (2001-07-05), pages 97-110, XP002234275
- DE LUZE A, SACHS L, DEMENEIX B.: "Thyroid hormone-dependent transcriptional regulation of exogenous genes transferred into Xenopus tadpole muscle in vivo. " PROC NATL ACAD SCI U S A. , vol. 90, no. 15, août 1993 (1993-08), pages 7322-7326, XP002253795
- SACHS LM, JONES PL, HAVIS E, ROUSE N, DEMENEIX BA, SHI YB.: "Nuclear receptor corepressor recruitment by unliganded thyroid hormone receptor in gene repression during Xenopus laevis development." MOL CELL BIOL., vol. 22, no. 24, décembre 2002 (2002-12), pages 8527-8538, XP002253796
- DATABASE BIOSIS [en ligne] BIOSCIENCES INFORMATION SERVICE, PHILADELPHIA, PA, US Juin 2002 ZOELLER R THOMAS ET AL: 'Thyroid hormone, brain development, and the environment' Database accession no. PREV200200399959
- DATABASE BIOSIS [en ligne] BIOSCIENCES INFORMATION SERVICE, PHILADELPHIA, PA, US Décembre 1999 FURLOW J DAVID ET AL: 'In vitro and in vivo analysis of the regulation of a transcription factor gene by thyroid hormone during Xenopus laevis metamorphosis' Database accession no. PREV200000049749
- DATABASE BIOSIS [en ligne] BIOSCIENCES INFORMATION SERVICE, PHILADELPHIA, PA, US 1993 FOSTER ROSEMARY ET AL: 'Multiple SW16-dependent cis-acting elements control SW14 transcription through the cell cycle' Database accession no. PREV199396040121
- DATABASE GENBANK [en ligne] FURLOW, J.D. AND BROWN, D.D.: 'Xenopus laevis TH/bZIP B gene promoter region, and exon 1' Database accession no. AF192492
- ERICA GIES: 'Unhealthy Glow: Fluorescent Tadpoles Expose Chemical Contamination' SCIENTIFIC AMERICAN, [en ligne] 07 Février 2013, Extrait de l'Internet: <URL:http://www.scientificamerican.com/arti cle/transgenic-tadpole-glows-to-reveal-chem ical-contamination/>

## Description

La présente invention se rapporte au domaine de la biologie, et plus particulièrement de la transgenèse animale. Plus particulièrement la présente invention se rapporte à des grenouilles transgéniques, notamment au stade tétard, ou les cellules dérivées de ceux-ci, ainsi que leur mise en oeuvre dans des procédés destinés à identifier la présence de perturbateurs endocriniens dans l'environnement, tels que les molécules agonistes ou antagonistes de récepteurs nucléaires.

Une grande variété de composés chimiques diffuse largement dans le milieu naturel. Parmi ceux-ci, nombreux sont ceux qui ont des effets de type hormonal indésirables du fait de leurs propriétés physio-chimiques. La présence de polluants hormonaux dans les eaux naturelles est ainsi révélée par l'observation de problèmes de fertilité chez différentes espèces aquatiques. De fait, plusieurs voies de recherches actuelles confirment la présence de perturbateurs endocriniens dans notre environnement.

Les substances perturbatrices du système endocrinien miment les effets biologiques de facteurs hormonaux (oestrogènes, androgènes et hormones thyroïdiennes) qui régulent finement de nombreuses fonctions telles que les fonctions d'homéostasie, de reproduction, de développement ou de comportement. Ces substances d'origine naturelle ou de synthèse, sont en fait reconnues par un organisme vivant comme des facteurs moléculaires intervenant dans les processus régulés par les hormones et interfèrent donc avec l'action des hormones.

La présence répandue de ces facteurs provoque l'attention des autorités gouvernementales à travers le monde et la nécessité pour les grandes entreprises de prendre en compte l'impact environnemental de leurs activités. En réponse à ces problèmes, il est nécessaire, voire stipulé légalement de tester tout produit chimique ou rejet industriel du point de vue de leurs activités hormonales.

Il est donc crucial de mettre en place une expertise fiable, basée sur la production de modèles intégrés, donnant des résultats sensibles, rapides, quantifiables, spécifiques des tissus et possédant une grande souplesse d'utilisation, de détection des perturbateurs endocriniens environnementaux. C'est le problème que se propose de résoudre la présente invention en fournissant des organismes aquatiques transgéniques de préférence au stade embryonnaire et donc transparent dans les cellules desquels l'expression d'une protéine rapporteuse est modulée de manière fine par la présence de facteurs perturbateurs de la fonction hormonale. En outre, la présente invention permet par la combinaison de plusieurs protéines rapporteuses, de préférence fluorescentes, de mesurer simultanément et de façon précoce dans le même animal différents polluants. Selon la construction génique utilisée, il est également possible de déceler des effets spécifiques d'un tissu (effets neurotoxiques, effets hépato-toxiques, ...).

La présente invention concerne un animal selon la revendication 1. En particulier, il est ici décrit une cellule transgénique d'animal aquatique sélectionné parmi les amphibiens et les téléostes comprenant au moins une cassette d'expression, la dite cassette comprenant une séquence d'ADN régulatrice, de préférence de vertébrés, sélectionnée parmi les éléments de réponse aux récepteurs nucléaires d'hormones, liée de manière opérationnelle en amont d'un segment d'ADN codant pour une protéine rapporteuse, et facultativement un signal de polyadénylation et facultativement une séquence "insulateur" à chaque extrémité de la dite cassette. Préférablement ladite cassette comprend en outre une séquence promotrice minimale.

On entend désigner ici par transgénique une cellule, ou un animal comportant au moins une cellule, comportant un transgène. On entend désigner par « transgène » ou par « séquence d'acides nucléiques exogène », ou par « gène exogène », ou par "cassette d'expression", termes qui seront indifféremment employés dans la présente demande, du matériel génétique qui a été ou qui va être inséré artificiellement dans le génome d'un animal, particulièrement dans une cellule d'animal cultivée *in vitro* ou dans une cellule d'animal vivant, ou qui va se maintenir dans la dite cellule sous forme épisomale. De manière préférée, le transgène comprend au moins une séquence susceptible d'être transcrite ou transcrite et traduite en protéine.
De préférence, mais de manière non exhaustive, les éléments de réponse aux récepteurs nucléaires d'hormones sont choisis parmi l'élément de réponse aux oestrogènes (ERE)(Mader et *al.* 1993), l'élément de réponse aux hormones thyroïdiennes (TRE) l'élément de réponse aux glucocorticoides (GRE) et l'élément de réponse aux minéralocorticoides (MRE) (Kolla et *al*.1999), l'élément de réponse à la progestérone (PRE) (Hecht et *al*.1988), l'élément de réponse à la vitamine D (DRE) (Kitazawa et *al.* 2002).

Préférentiellement l'élément de réponse aux récepteurs nucléaires d'hormones est le TRE. Différentes séquences régulatrices contenant des TRE peuvent être utilisées. Par exemple, la séquence TRE de l'enzyme malique de rat (Seq ID N°1), la séquence TRE consensuelle DR4 en un exemplaire (Seq ID N°5 ; Seq ID N°6), la séquence TRE consensuelle en triple exemplaire (DR4)X3, les séquences DR4 étant espacées de 4 nucléotides. Alternativement, l'élément de réponse aux récepteurs nucléaires d'hormones est le ERE.

Par séquence régulatrice ou éléments de régulation de l'expression du gène, on entend désigner toutes les séquences d'ADN impliquées dans la régulation de l'expression génique c'est-à-dire essentiellement les séquences régulatrices de la transcription, de l'épissage, de la traduction. Parmi les séquences d'ADN régulatrices de la transcription, il convient de citer la séquence promotrice minimale, les séquences amonts (par exemple, les éléments de réponse aux récepteurs nucléaires d'hormones tels TRE, ERE, GRE,...), les séquences activatrices (« enhancers »), éventuellement les séquences inhibitrices (« silencers »), les séquences insulateurs (« insulator »), telles les MAR (Matrix Attachment Region) (Seq ID N°10, N°11), les séquences d'épissage. La séquence d'ADN régulatrice est de préférence une séquence de vertébrés, de manière préférée une séquence de xénope ou de téléostes, et de manière plus préférée une séquence humaine.

Ces séquences de régulation de l'expression sont liées de manière opérationnelle au(x) gène(s) rapporteur(s). Une séquence nucléique est « liée de manière opérationnelle » lorsqu'elle est placée dans une relation fonctionnelle avec une autre séquence d'acide nucléique. Par exemple, un promoteur ou une séquence régulatrice amont est liée de manière opérationnelle à une séquence codante, s'il module ou affecte la transcription de ladite séquence codante. Concernant les séquences régulatrices de la transcription, « lié de manière opérationnelle » signifie que les séquences d'ADN liées sont contiguës, et lorsqu'il s'agit de lier deux régions codantes pour des protéines, contiguës et en phase de lecture.

Par séquence promotrice minimale, on entend désigner un promoteur qui dépourvu de ses séquences régulatrices amonts est capable d'induire la transcription du gène, auquel elle est opérationnellement liée à un niveau basal. A titre d'exemple de promoteur minimal, il convient de citer le promoteur du gène de la thymidine kinase du virus de l'herpès simple, le promoteur du cytomégalovirus simien (simian CMV IE94), le promoteur E1b d'adénovirus (Argenton et *al.* 1996).

Les séquences promotrices et régulatrices sont définies en fonction du profil d'expression désiré de la protéine rapporteuse. De préférence, le gène rapporteur est placé sous le contrôle d'éléments d'expression tissus-spécifiques ou cellules-spécifiques ou ubiquitaires.

Les éléments d'expression tissus-spécifiques ou promoteurs tissus-spécifiques sont choisis parmi les promoteurs qui permettent d'obtenir une expression spécifique, et de préférence forte, dans une ou plusieurs cellule(s), tissu(s), type(s) cellulaire(s), ou organe(s) de l'animal aquatique. Ces promoteurs peuvent être ou non hétérologues à l'organisme et être naturellement présents ou non dans le génome de l'organisme. Les promoteurs tissus-spécifiques selon l'invention sont de préférence choisis parmi les séquences qui dirigent l'expression dans le foie, le système nerveux central, notamment l'hypothalamus, et les ostéoblastes. L'expression dans le foie étant particulièrement intéressante étant donné que les polluants environnementaux se trouvant concentrés dans cet organe de détoxification. A ce propos, on peut citer les promoteurs des gènes :
- de la vitallogénine, CYP26, CYP3A qui dirigent l'expression du gène dans le foie. Le promoteur du gène de la vitellogénine de xénope et de poulet est un exemple de promoteur tissu-spécifique. L'expression de ce gène dans le foie est induite par les oestrogènes. Le gène de la vitellogénine A2 de xénope contient un élément de réponse aux oestrogènes compris entre -331 et -319. Le gène de la vitellogénine de poulet contient différents éléments de réponse aux hormones telles les glucocorticoides, les oestrogènes et les progestines situés entre -721 et -591 pb.
- le promoteur de stromélysine 3 qui code un enzyme participant au remodela ge de la matrice extracellulaire s'exprimant dans les bourgeons de membres, l'intestin au cours de la métamorphose, dans certains tissus en remodelage (Ishizuya-Oka et *al.* 1996).
- de la vasopressine, BTEB, TH/bZIP qui dirige l'expression du gène dans le cerveau. La vasopressine est une hormone neuro-hypophysaire impliquée dans le métabolisme de l'eau et la régulation de la pression sanguine ; elle est également impliquée dans la neurotransmission et la neuro-modulation dans le système nerveux central. L'expression de ce gène est régulée par les oestrogènes et la testostérone. La séquence promotrice de la vasopressine de rat contient deux éléments de réponse aux oestrogènes (ERE) situés dans un fragment distal de 1,5 kbp (Shapiro et *al.,* 2000). Le promoteur du gène BTEB (Basic Transcription Element-Binding) constitue un autre exemple de promoteur tissu-spécifique (Denvers et al., 1997). Le gène BTEB s'exprime dans le cerveau et son expression est induite par l'hormone thyroidienne T3 in vivo (Denvers et al., 1999). Une partie du promoteur du gène BTEB de xénope a été cloné (Brown et *al.,* 1996). Un autre exemple de promoteur tissu-spécifique est constitué par le promoteur (Seq ID N°12) du gène TH/bZIP qui code pour un facteur de transcription à domaines basiques et leucine zipper (Wang et *al.,* 1993). L'expression du gène TH/bZIP est effectivement régulée par les hormones thyroidiennes exogènes et au moment de la métamorphose du xénope qui est un processus dépendant des hormones thyroidiennes (Furlow et al., 1999). La portion de promoteur du gène TH/bZIP comprise entre - 246 pb et +130 pb contient deux TRE (Seq ID N°2 et N°3).
- Cbf1 qui code un facteur de transcription spécifiquement exprimé dans les ostéoblastes (Kasenty et al., 2000). L'expression de ce facteur qui joue un rôle déterminant dans la différenciation et la fonction des ostéoblastes, est induite par les oestrogènes et son promoteur contient un ERE.
Dans le cadre d'une cellule ou d'un animal multitransgénique tel que divulgué ici il est possible d'analyser simultanément une régulation positive ou négative de l'expression du gène rapporteur. Par exemple, le promoteur de la TSH (Thyroid stimulating hormone) en amont de la GFP est régulé positivement par l'hormone T3, alors que le promoteur de la TRH (Thyrotropin releasing hormone) est régulé négativement par la T3. Ainsi une cellule comprenant les cassettes d'expression « promoteur de la TSH/GFP » et « promoteur de la TRH/RFP) permettrait d'identifier ou de cribler des composés agissant positivement ou négativement sur l'expression génique.
L'article de Metzger et Feil (1999) donne à titre d'exemple non limitatif (cf. tableau page 471) une liste de promoteurs tissus-spécifiques susceptibles d'être utilisés pour diriger l'expression de la protéine rapporteuse dans différents tissus. Les promoteurs tissus-spécifiques sont d'une manière plus générale choisis parmi ceux qui dirigent l'expression de la protéine rapporteuse dans un système physiologique, un organe, un tissu, un type cellulaire, ou une cellule particulière, parmi lesquels il convient de citer de manière non exhaustive le système nerveux en général, et notamment le cerveau, le cervelet, les neurones, les motoneurones, les cellules gliales, les cellules de Schwann, l'hypophyse, l'hypothalamus, la glande pituitaire, l'hippocampe et le cortex, le coeur, les cardiomyocytes ventriculaires et les cardiomyocytes auriculaires, les poumons, les os, les yeux et plus particulièrement la rétine et le cristallin, la peau et plus particulièrement le derme et l'épiderme, les muscles, et plus particulièrement les muscles squelettiques, le muscle cardiaque, les muscles lisses, la glande mammaire, les gonades et plus particulièrement les testicules, les ovaires, les cellules germinales, les ovocytes, les ovogonies, les spermatozoïdes, les spermatogonies et les spermatocytes, le rein, le foie et notamment les hépatocytes, la rate, le pancréas et notamment les cellules de Langerhans et les cellules β, la langue, l'oesophage, le tissu adipeux, les cellules endothéliales.

Les éléments d'expression ubiquitaire ou promoteurs ubiquitaires sont choisis parmi les promoteurs qui permettent d'obtenir une expression, de préférence forte, dans l'ensemble, ou pour le moins dans une grande proportion d'organes, ou de tissus de l'organisme divulgué ici. Ces promoteurs peuvent être hétérologues ou non à l'organisme selon l'invention. A titre d'exemple non limitatif de promoteurs ubiquitaires, on peut citer le promoteur du cytomégalovirus (CMV) (Schmidt et *al.,* 1990) et le promoteur inductible par l'interféron (Mx1) (Hug et *al.,* 1998; Arnheiter et *al.,* 1990). En outre, les éléments d'expression, ou promoteurs, peuvent assurer un contrôle constitutif ou inductible de l'expression du gène de fusion. Parmi les éléments assurant une expression inductible, il convient de citer les promoteurs eucaryotiques inductibles par les métaux lourds (Mayo et *al.,* 1982; Brinster et *al.,* 1982; Seark et *al.,* 1985), par un choc thermique (Nover *et al.,* 1991), par les hormones (Lee *et al.,* 1981; Hynes et *al.,* 1981; Klock *et al.,* 1987; Israel *et al.,* 1989), par l'interféron (Hug *et al.,* 1998); Arnheiter *et al.,* 1990). Il convient également de citer les éléments d'expression procaryotiques inductibles tels que le système répresseur Lac (LacR/opérateur/inducteur) d'E. coli (Hu et *al.,* 1987 ;Brown et *al.,* 1987; Figge *et al.*, 1988; Deuschle et *al.,* 1990; Labow *et al.,* 1990), le système de résistance à la tétracycline d'*E*. *coli* (Gossen *et al.,* 1992)(WO 94 04 672, EP 804 565).

La dite protéine rapporteuse est sélectionnée dans le groupe des protéines auto-fluorescentes et des enzymes détectables par un procédé histo-chimique. De préférence, la dite protéine fluorescente est choisie dans le groupe composé de la protéine de fluorescence verte (GFP), le protéine de fluorescence verte augmentée (EGFP), le protéine de fluorescence rouge (CFP et Red FP, RFP), la protéine de fluorescence bleue (BFP), la protéine de fluorescence jaune (YFP), et les variants fluorescents de ces protéines, les protéines fluorescentes changeant de couleur avec le temps (« fluorescent timer™ commercialisée par Clonetech »), les protéines fluorescentes dont la fluorochrome a une durée de vie très courte, les protéines de fusion, telles la protéine de fusion luciférase/GFP composée d'au moins deux protéines fluorescentes et qui permettent une anayse visuelle et quantitative. Parmi les protéines fluorescentes utilisées, la protéine changeant de couleur avec le temps est particulièrement appréciée, car le changement de fluorescence verte vers une fluorescence rouge reflète l'activité du promoteur qui dirige l'expression de cette protéine rapporteuse. Il est ainsi possible d'évaluer la persistance d'un composé ou d'un polluant sur l'activité du promoteur, et ce après le contact de l'animal selon l'invention avec le polluant. L'utilisation d'un tel type de protéine rapporteuse permet ainsi d'évaluer la dynamique d'activation d'un promoteur. Selon un autre type de réalisation, il est intéressant d'utiliser des protéines dont la fluorescence est de courte durée de vie. Ainsi, l'examen des cellules et des animaux sera immédiat après l'arrêt de la mise en contact avec le ou les polluants. La disparition de la fluorescence ou sa persistance après un temps supérieur à celui de la demi vie du fluorochrome indiquera si le ou les promoteurs sont encore activés. Selon un autre type de réalisation, il est intéressant d'utiliser un gène rapporteur codant pour une protéine de fusion composée de deux protéines fluorescentes différentes réunies par un peptide de liaison qui contient un site de reconnaissance de la caspase-3 (Xu et *al*., 1998 et Luo et al., 2001). Ainsi, les séquences codant pour la GFP et BFP, respectivement CFP et YFP ont été réunies par un peptide de 18 acides aminés contenant une séquence de clivage spécifique de la caspase 3, une des protéines effectrices de la mort cellulaire programmée. Il existe un transfert d'énergie entre les deux fluorophores tant que les protéases caspase-3 ne sont pas activées. L'entrée en apoptose des cellules active les caspase-3 avec, pour conséquence, le clivage de la protéine de fusion et arrêt du transfert d'énergie, et donc la visibilité de la GFP, respectivement de la CFP. Une telle cellule ou animal transgénique permet donc de détecter l'effet du polluant sur le phénomène d'apoptose. L'utilisation de cellules ou d'animaux transgéniques comportant deux gènes rapporteurs codant pour les protéines de fusion auto-fluorescentes GFP/BFP et CFP/YFP permet d'obtenir simultanément le même type d'information sur une éventuelle toxicité de deux catégories distinctes de polluants (oestrogènes et hormones tyroïdiennes par exemple). De telles cellules ou animaux transgéniques sont particulièrement utiles dans le cas de deux transgènes répondant à des types hormonaux différents mais s'exprimant dans les mêmes tissus ou bien d'expression ubiquitaire.

Selon un autre mode de réalisation, l'enzyme détectable par un procédé histo-chimique est choisie dans le groupe composé de la luciférase, la β-galactosidase, la β-glucuronidase, la phophatase alcaline, la chloramphénicol-acétyl-transférase, la déshydrogénase alcoolique. Selon un mode préféré de réalisation, il s'agit de la luciférase. Par luciférase, on entend désigner toutes les protéines qui catalysent ou initient une réaction bioluminescente en présence d'un substrat appelé luciférine. La luciférase selon l'invention peut provenir de nombreux organismes ou systèmes générant de la bioluminescence (voir US 6,152,358). Par exemple, la luciférase selon l'invention peut provenir de *Renilla* (US 5,418,155 et US 5,292,658) ou de *Photinus pyralis,* ou de *Luciola cruciata* (US 4,968,613).

Les technologies de l'ADN recombinant utilisées pour la construction de la cassette d'expression ou le vecteur d'expression la contenant, sont celles connues et communément utilisées par les hommes de l'art. Les techniques standard sont utilisées pour le clonage, l'isolement de l'ADN, l'amplification, et la purification ; les réactions enzymatiques impliquant l'ADN ligase, l'ADN polymérase, les endonucléases de restriction sont effectuées selon les recommandations du fabricant. Les vecteurs incluent des plasmides, les cosmides, les phagemides, les bactériophages, les rétrovirus et autres virus animaux, les chromosomes artificiels, tels les YAC, BAC, HAC et autres vecteurs analogues. Ces techniques et les autres sont généralement réalisés selon Sambrook et al. (1989 Molecular cloning: a laboratory manual second édition - Cold Spring Harbor Laboratory Press. Cold Spring Harbor, NY. USA).

La présente divulgation concerne couvre également la cassette d'expression ou transgène selon l'invention, ainsi que les vecteurs les contenant. En effet, le transgène peut être cloné dans un vecteur de clonage qui permet d'en assurer sa propagation dans une cellule hôte, et/ou facultativement dans un vecteur d'expression pour assurer l'expression du transgène.

Les méthodes pour générer des cellules transgéniques sont bien connues de l'homme de l'art. Pour introduire ladite cassette d'expression ou ledit transgène, facultativement compris dans un vecteur linéarisé ou non, ou sous la forme d'un fragment de vecteur, peut être introduit dans la cellule hôte par des méthodes standard telles que par exemple la micro-injection dans le noyau (US 4 873 191), la transfection par précipitation au phosphate de calcium, la lipofection, l'électroporation, le choc thermique, la transformation avec des polymères cationiques (PEG, polybrène, DEAE-Dextran...), l'infection virale, le sperme (Kroll et Amaya,1996). Kroll et Amay1999) ont plus particulièrement décrit un procédé pour générer des embryons de grenouilles transgéniques. Pour réaliser le criblage des cellules dans lesquelles au moins un transgène a été introduit, des marqueurs positifs et/ou négatifs, encore appelés gènes de sélection, peuvent être insérés dans le vecteur. Différents systèmes de sélection des cellules ont été décrits ; il convient de citer le système décrit qui utilise des vecteurs de sélection positif/négatif (Capecchi et *al.,* 1989).

Selon un premier mode de réalisation, la cellule comprend une cassette d'expression comprenant de manière séquentielle dans le sens 5' - 3', la séquence du TRE de l'enzyme malique de rat (Seq ID N°1), le promoteur minimal du gène de la thymidine kinase (TK) du virus de l'herpès simplex lié de manière opérationnelle à un segment d'ADN codant pour la protéine de fluorescence verte augmentée, un signal de polyadénylation et facultativement une séquence "insulateur" à chaque extrémité de la dite cassette (Seq ID N°10 ou Seq ID N°11).

Selon un second mode de réalisation, la cellule comprend de manière séquentielle dans le sens 5' - 3', la séquence promotrice du gène de la stromélysine 3 de xénope comprenant plusieurs TRE (Seq ID N°7, Seq ID N°8, Seq ID N°9), liée de manière opérationnelle à un segment d'ADN codant pour la luciférase, un site de polyadénylation et facultativement une séquence « insulateur » à chaque extrémité de ladite cassette (Seq ID N°10 ou N°11).

Selon un troisième mode de réalisation, la cellule comprend de manière séquentielle dans le sens 5' - 3', la séquence du TRE de l'enzyme malique de rat (Seq ID N°1), le promoteur minimal du gène de la thymidine kinase (TK) du virus de l'herpès simplex lié de manière opérationnelle à un segment d'ADN codant pour la luciférase, un signal de polyadénylation et facultativement une séquence "insulateur" à chaque extrémité de la dite cassette (Seq ID N°10 ou N°11).

La cellule d'animal aquatique et/ou l'animal transgénique non humain est obtenue en introduisant au moins un cassette d'expression codant pour une protéine rapporteuse, dans une cellule, un zygote ou un embryon précoce de l'animal. L'introduction de différents transgènes dans la cellule peut également être réalisée de manière simultanée ou de manière décalée dans le temps. Lorsque la cellule est multitransgénique alors, de préférence, les transgènes codent chacun pour une protéine rapporteuse distincte, et l'expression de chaque protéine rapporteuse est spécifique d'un type de polluants environnementaux.

Ladite cellule comprend au moins un transgène tel que décrit plus haut, présent soit sous forme d'élément extra-chromosomal, soit intégrée stablement de manière aléatoire ou ciblée dans l'ADN chromosomique. Dans le cas où plusieurs transgènes sont présents dans la cellule selon l'invention, ceux-ci peuvent être présents sous forme d'élément extra-chromosomal et/ou intégré stablement de manière aléatoire ou ciblée dans l'ADN chromosomique.

Selon un mode préféré de réalisation, la cassette d'expression est intégrée stablement, de manière aléatoire ou ciblée, dans l'ADN chromosomique. Par intégration de manière stable, on entend signifier l'insertion du transgène dans l'ADN génomique de la cellule selon l'invention. Le transgène ainsi inséré est ensuite transmis à la descendance cellulaire.

Alternativement, la cellule est caractérisée en ce que la dite cassette d'expression ou transgène codant pour au moins une protéine rapporteuse est présent sous forme épisomale dans ladite cellule. Il est à la portée de l'homme du métier de définir la nature et les caractéristiques du vecteur d'expression utilisé pour permettre le maintien et l'expression sous forme épisomale du transgène dans la cellule.

Dans le cadre de la transgenèse germinale, l'ensemble des cellules de l'animal et notamment ses cellules de la lignée germinale sont transgéniques. Dans ce cas la cassette d'expression est de préférence intégrée stablement, de manière aléatoire ou ciblée, dans l'ADN chromosomique. Dans le cas où l'intégration du gène rapporteur est ciblée par recombinaison homologue dans le génome de l'organisme (« Knock-in »), le gène rapporteur peut être dépourvu de promoteurs et/ou d'éléments d'expression et être placé sous le contrôle d'un promoteur ou d'éléments d'expression endogènes.

Dans le cadre de transgenèse somatique, seule une partie des cellules de l'animal sont transgéniques, au niveau du site d'injection du transgène. Dans ce cas la cassette d'expression ou transgène est présent de préférence sous forme épisomale dans la cellule.

Ladite cellule est de préférence une cellule de d'amphibien, de préférence la grenouille sélectionnée parmi *Xénopus laevis* et *Xénopus tropicalis.* Compte tenu des polymorphismes génétiques présents dans la population, il peut être intéressant pour analyser ou obtenir une réponse physiologique ou comportementale caractéristique que les amphibiens transgéniques, et notamment les grenouilles transgéniques présentent des fonds génétiques différents telles que les espèces *Xénopus laevis, Xénopus tropicalis.* Selon un autre mode de réalisation, la cellule est une cellule de téléoste de préférence du zebra fish (poisson zèbre) ou du médaka.

La présente invention se rapporte également à un animal aquatique transgénique non humain sélectionné parmi les amphibiens et les téléostes, comprenant au moins une cellule telle que précédemmennt décrite. Lorsque cet animal est un animal aquatique/terrestre comme les amphibiens ou batraciens, il est sélectionné parmi les anoures, les urodèles et les apodes. De préférence il s'agit d'anoures, de manière plus préférée de xénopes des familles pipidae et ranidae. Les xénopes sont de préférence sélectionnés parmi *Xénopus laevis* et *Xénopus tropicalis.* Selon un second mode de réalisation de l'invention, l'animal est un téléoste. Par téleostes, on entend désigner des poissons « osseux », c'est-à-dire que leur squelette est totalement ossifié. Le groupe des téléostes inclut par exemple le poisson zèbre (« zebrafish »), le medaka, le rerio géant, le « puffer fish ».

Les animaux aquatiques de la présente invention présentent un certain nombre d'avantage par rapport aux systèmes d'animaux modèles traditionnels tels la souris, la drosophile ou le nématode. Tout d'abord, le xénope et les téléostes sont d'un point de vue évolutif plus proche de l'homme, et à ce titre le modèle de criblage de la présente invention est beaucoup plus relevant. Les bases moléculaires et morphologiques du développement des tissus et des organes est soit identique soit similaire aux autres vertébrés, dont l'homme. Le deuxième avantage procuré par les animaux aquatiques modèles de la présente invention, est que leurs embryons sont très transparents. Etant donné la transparence des embryons, l'activité des composés administrés ou mis en contact avec l'animal, peut être détectée et diagnostiquée beaucoup plus rapidement que chez les animaux non transparents. Ces activités ne peuvent être détectée essentiellement qu'au stade embryonnaire chez le xénope. Un autre avantage de ces animaux aquatiques est qu'ils se développent rapidement en comparaison des animaux comme la souris. Les animaux aquatiques selon l'invention présentent également l'avantage que les composés à tester peuvent être administrés directement sur l'animal en développement, ce qui n'est pas le cas pour les animaux qui se développent *in vitro.* Enfin, un autre avantage non négligeable, du choix de l'animal aquatique selon l'invention est le coût de la maintenance et de l'élevage qui est faible comparativement à celui généré par des animaux tels la souris. Enfin, compte tenu de la petite taille de l'embryon du xénope ou du zebrafish, le système selon l'invention se prète bien à l'utilisation de microplaques multi-puits pour la détection en grande quantité de l'expression du gène rapporteur, et permettre ainsi une automatisation de cette détection.

Plus particulièrement l'invention couvre une grenouille transgénique et ses descendants, aux différents stades de leur développement, et de préférence au stade tétard, obtenue par trangenèse germinale caractérisée en ce que l'ensemble des cellules de l'animal sont transgéniques. Par différents stades du développement on entend désigner le zygote, le tétard, la grenouille adulte.

Selon un autre mode de réalisation, l'invention couvre la grenouille transgénique, au différents stade de son développement, et de préférence au stade tétard, obtenue par trangenèse somatique caractérisé en ce que une partie des cellules du dit animal sont transgéniques.

Il est évident que l'animal transgénique peut comprendre des cellules transgéniques issues de la transgenèse germinale et des cellules transgeniques issues de la transgenèse somatique.

De manière plus préférée, la grenouille transgénique selon l'invention est au stade tétard. Selon un premier exemple, la présente divulgation couvre un tétard transgénique comprenant au moins une cellule comprenant une cassette d'expression comprenant de manière séquentielle dans le sens 5'-3' la séquence promotrice du gène de la stromélysine 3 de xénope comprenant plusieurs TRE (Seq iD N°7, N°8, N°9), liée de manière opérationnelle à un segment d'ADN codant pour la luciférase, un signal de polyadénylation et facultativement une séquence "insulateur" à chaque extrémité de la dite cassette. Selon un second exemple, la présente divulgation, couvre un tétard transgénique comprenant au moins une cellule comprenant une cassette d'ADN d'expression comprenant de manière séquentielle dans le sens 5'-3' la séquence du TRE de l'enzyme malique de rat (Seq ID N°1), le promoteur minimal du gène de la thymidine kinase (TK) du virus de l'herpès simplex lié de manière opérationnelle à un segment d'ADN codant pour la luciférase, un signal de polyadénylation et facultativement une séquence "insulateur" à chaque extrémité de la dite cassette. Selon un troisième exemple,la présente divulgation couvre un tétard transgénique comprenant au moins une cellule comprenant une cassette d'ADN d'expression comprenant de manière séquentielle dans le sens 5'-3' la séquence du TRE de l'enzyme malique de rat (Seq ID N°1), le promoteur minimal du gène de la thymidine kinase (TK) du virus de l'herpès simplex lié de manière opérationnelle à un segment d'ADN codant pour la protéine de fluorescence verte augmentée,un signal de polyadénylation et facultativement une séquence "insulateur" à chaque extrémité de la dite cassette.

C'est également un des objets de la présente invention de fournir un procédé pour identifier la présence d'au moins un polluant environnemental qui module, c'est-à-dire inhibe ou stimule (de préférence stimule), la transcription médiée par des éléments de réponse aux récepteurs nucléaires d'hormones, comprenant les étapes de :
a) mise en contact d'un animal selon l'invention, dans un milieu aqueux comprenant le polluant environnemental ;
b) mise en contact d'un animal selon l'invention dans le milieu aqueux;
(c) détermination qualitative, facultativement quantitative, de l'expression de la protéine rapporteuse en a) et b) puis comparaison desdites expressions ;
de sorte qu'une différence d'expression de la dite protéine rapporteuse en a) et b) indique la présence de polluants environnementaux dans le milieu. Le procédé se caractérise en ce que à chaque type de polluant environnemental correspond une protéine rapporteuse distincte.

C'est également un objet de la présente invention de fournir un procédé de criblage de composé(s) modulant c'est-à-dire inhibant ou stimulant (de préférence stimulant), la transcription médiée par des éléments de réponse aux récepteurs nucléaires d'hormones, comprenant les étapes de :
a) mise en contact d'un animal selon l'invention, dans un milieu aqueux comprenant le ou les composés ;
b) mise en contact d'un animal selon l'invention, dans le dit milieu aqueux;
(c) détermination qualitative, facultativement quantitative, de l'expression de la protéine rapporteuse en a) et b) puis comparaison desdites expressions ;
(d) sélection du ou des composés induisant une différence d'expression de la dite protéine rapporteuse en a) et b).

Le procédé de criblage selon l'invention peut comprendre en outre l'étape de transfert somatique dans la cellule ou dans au moins une cellule transgénique de l'animal selon l'invention, d'au moins un gène de récepteur nucléaire d'hormones.En effet, l'adjonction d'un transgène sur exprimant au moins un récepteur nucléaire d'hormones dans une cellule de l'animal selon l'invention permet d'augmenter la sensibilité de détection lorsque les procédés de l'invention sont mis en oeuvre. De tels transgènes comportent des promoteurs tissus spécifiques ou ubiquitaire pour diriger l'expression du dit récepteur aux endroits appropriés.

Le procédé est également destiné au criblage simultané d'au moins deux composés caractérisé en ce que à chaque composé correspond une protéine rapporteuse distincte.

Le procédé selon l'invention est particulièrement utile pour déterminer si un composé connu est susceptible d'être un perturbateur endocrinien. Pratiquement, le composé à tester susceptible d'avoir un effet sur la transcription médiée par des éléments de réponse aux récepteurs nucléaires d'hormones est mis en présence de la cellule ou de l'animal aquatique en ajoutant directement le dit composé dans le milieu aqueux contenant la cellule ou l'animal vivant. En pratique, si il s'agit d'une cellule, le ou les dits composés à tester sont placés dans le milieu de culture. Si il s'agit d'un animal, notamment d'un tétard, le ou les composés à tester sont placés dans le milieu aqueux dans lequel vit l'animal. Alternativement si il s'agit de détecter la présence de composés dans l'eau d'une rivière, d'un lac ou de tout autre milieu aquatique naturel dans lequel ont suspecte la présence de contaminants environnementaux, l'animal est placé dans ce milieu aquatique. Dans ce cas, le milieu aqueux servant de contrôle pour mettre en oeuvre l'étape b) des procédés selon l'invention, peut être l'eau de la rivière, du lac ou de tout autre milieu aquatique naturel traité ou non et ne contenant pas le composé à tester ou à cribler. De telles approches sont utilisées pour introduire des agents chimiques dans les embryons de poissons (M. Westerfield, The zebrafish book : a guide for the laboratory use of zebrafish 3rd Ed., 1995). Alternativement les composés peuvent être administrés aux animaux aquatiques selon l'invention par électroporation, lipofection, ou ingestion. Alternativement, le ou les composés à cribler ou à détecter peuvent être directement mis en contact avec l'animal selon l'invention en le ou les injectant directement dans l'animal vivant. Par exemple, le composé peut être injecté dans le cerveau ou le muscle dorsal d'un tétard.

Le composé à cribler ou à étudier peut être administré seul ou en conjonction avec d'autres composés ou solvants ou transporteurs. Le composé peut être mis en présence de l'animal simultanément, au même moment ou après l'administration du réactif ou du colorant utilisé pour la détection de l'expression du gène rapporteur.

Il peut être intéressant avant de mettre en oeuvre les procédés selon l'invention de prétraiter animaux selon l'invention pour augmenter leur sensibilité aux composés à cribler, détecter ou mesurer. Ainsi les inventeurs ont développé un procédé de traitement à base de perchlorate. Les animaux ou cellules sont traités au perchlorate pendant une durée pouvant allée jusqu'à 1 mois à environ 1 g/l dans le milieu aquatique ou le milieu de culture respectivement. Les animaux ou cellules sont ensuite sensiblisés aux composés (si il est connu).

Les composés susceptides d'être criblés par le procédé selon l'invention sont extrèmement variés et se caractérisent par leur aptitude à lier directement ou indirectement le récepteur nucléaire d'hormones. Le dit composé peut ainsi entrer en compétition avec le dit récepteur nucléaire pour sa liaison aux éléments de réponse (RE) aux récepteurs nucléaires d'hormones, ou bien avec le ligand du récepteur, c'est-à-dire l'hormone. Les composés sont des molécules agonistes ou antagonistes des récepteurs nucléaires tels que le récepteur aux hormones thyroidiennes (TR), le récepteur aux oestrogènes (ER), le récepteur à l'acide rétinoïque (RAR), le récepteur aux glucocorticoïdes (GR), le récepteur aux minéralocorticoïdes (MR), les récepteurs orphelins, le récepteur à la vitamine D. Le terme composé inclut tout élément, entité, agent incluant sans limitation ni exhaustivité, les polluants ou composés pharmaceutiques, thérapeutiques, pharmacologiques, chimiques, environnementaux, agricoles, aquatiques, cosmétiques, les drogues, les médicaments, les produits naturels ou synthétiques. Par exemple selon un mode préféré de réalistaion, le dit composé criblé possède un effet ostéoprotecteur dépourvu d'effets utérotrophiques indésirables et constitue donc un agent pharmaceutique.

Les techniques et moyens de détection de l'expression du ou des gènes rapporteur sont bien connues de l'homme du métier. Ils comprennent de manière non exhaustive, l'oeil humain, le film photographique, le photomultiplicateur, la photodiode (pour revue voir US 5 571 722). Les moyens de détection peuvent en outre comprendre un dispositif de mesure et/ou un ordinateur pour stocker les données et calculer le taux d'expression du gène rapporteur, et par comparaison avec un étalon, calculer la concentration dudit composés ou polluant environnemental dans le milieu. Plus généralement, les moyens de détection de la bioluminecence mesurent l'intensité de la lumière en fonction de la concentration en composés ou polluants environnementaux.

L'invention concerne l'utilisation du procédé selon l'invention pour l'analyse et l'étude du mode d'action biologique de composé(s) modulant la transcription médiée par des éléments de réponse aux récepteurs nucléaires d'hormones. L'invention concerne également l'utilisation du procédé selon l'invention pour étudier l'effet de dose des polluants environnementaux qui stimulent la transcription médiée par des éléments de réponse aux récepteurs nucléaires d'hormones.

Plus généralement c'est un objet de la présente invention d'utiliser un animal selon l'invention, pour la détection, l'analyse et/ou l'étude des contaminants environnementaux, notamment dans l'eau. L'invention est particulièrement utile pour analyser et/ou évaluer la qualité de l'eau.

Une autre utilisation de l'animal selon l'invention réside dans l'étude de toxicité et/ou de toxicologie, notamment en cosmétologie, en agrochimie, en pharmacie. Ainsi, la présente invention est particulièrement utile dans un protocole d'étude par FETAX.

Les animaux transgéniques selon l'invention sont également particulièrement utiles pour détecter, étudier et mesurer la léthalité cellulaire induite par certains composés, la tératogénicité et la tumorigénicité de certains composés, ainsi que les propriétés pro-apoptotique de certains composés.

L'invention vise également à protéger un dispositif pour la détection de polluants environnementaux, notamment dans l'eau, et/ou de composé(s) modulant la transcription médiée par des éléments de réponse aux récepteurs nucléaires d'hormones caractérisé en ce qu'il comprend au moins un animal, de préférence un xénope au stade tétard ou un zebrafish, selon l'invention, et facultativement des moyens de détection de l'expression de ou des gènes rapporteur.

D'autres caractéristiques et avantages de la présente invention seront mieux mis en évidence à la lecture des exemples suivants. Dans ces exemples on se réfèrera aux figures suivantes.

### FIGURES

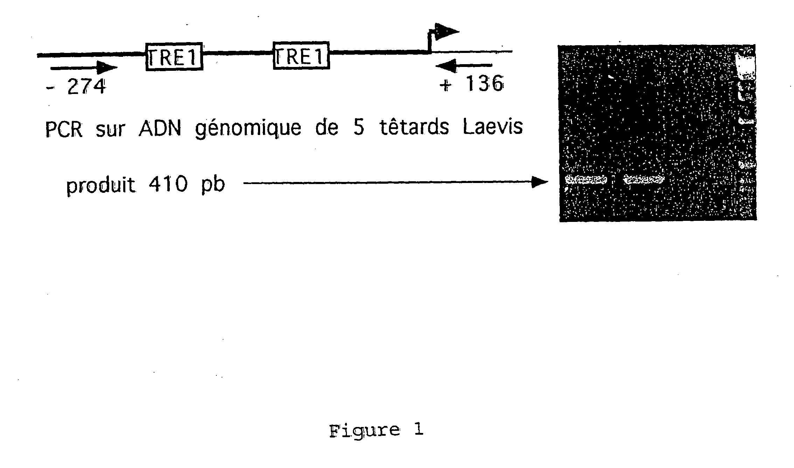

**Figure 1** : Représentation schématique du promoteur du gène TH/bZIP et de la position des amorces pour isoler par PCR, le fragment d'ADN contenant les deux éléments de réponses aux hormones thyroïdienne (TRE) le produit d'amplification de 410 pb est visualisé sur gel d'agarose.

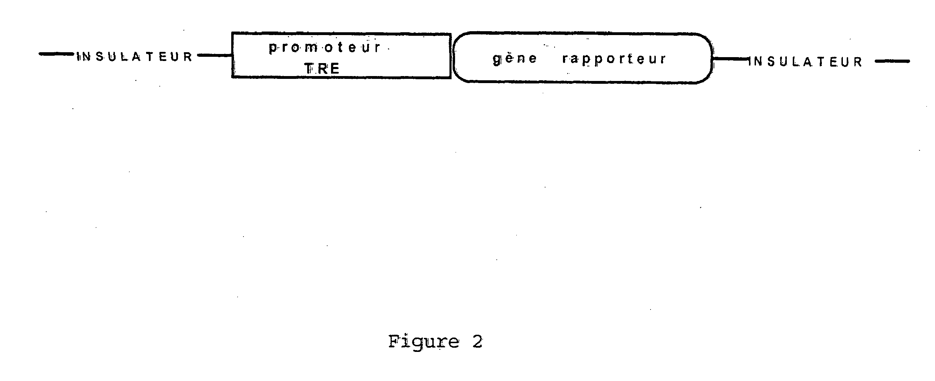

**Figure 2****:** Représentation schématique d'une cassette d'expression contenant une séquence promotrice contenant des TRE (Thyroid Responsive Element).

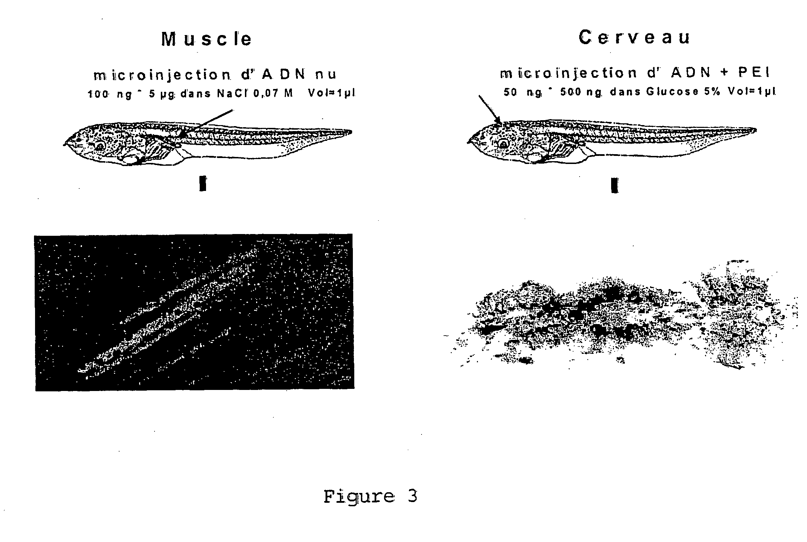

**Figure 3** : Transgenèse somatique dans le muscle (à gauche) et dans le cerveau (à droite). Dans le muscle, l'ADN est introduit dans les cellules par microinjection d'ADN nu. Dans le cerveau l'ADN est compléxée à du polyéthylènimine (PEI), puis micro injecté dans le cerveau.

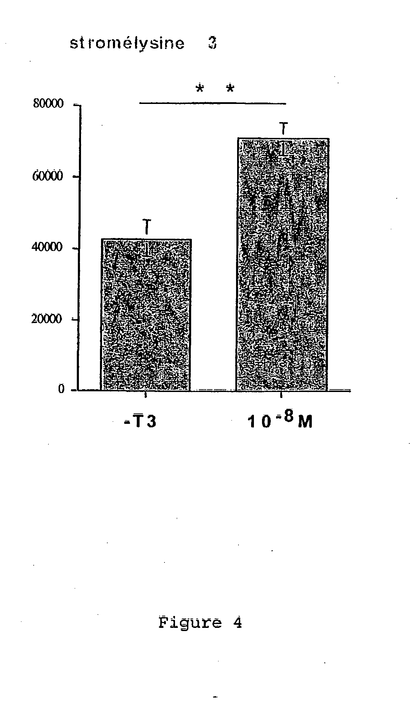

**Figure 4** : Test de fonctionnalité de la construction « promoteur de la stromélysime 3 de xénope couplée à la luciférase ». La construction est transfectée dans les cellules XTC en complexant l'ADN avec du PEI (22 kDA, à 6 équivalents), les cellules transfectées sont cultivées en présence (10⁻⁸ M) ou en absence (-T3) d'hormone thyroïdienne T3.

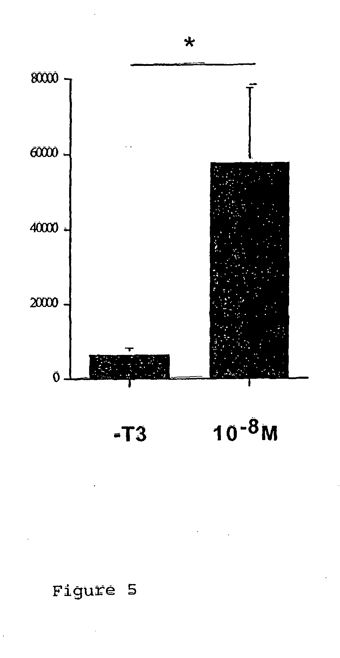

**Figure 5** : Transgenèse somatique par injection dans le muscle dorsal de xénope la construction TRE-tk-luciférase. Les animaux sont transfectés par injection dans le muscle dorsal de 1 mg/animal de la construction TRE-tk-luciférase. Les animaux sont ensuite traités avec (10⁻⁸ M) ou sans (-T3) hormone thyroïdienne T3. 4 jours après le traitement, l'expression de la luciférase est mesurée.

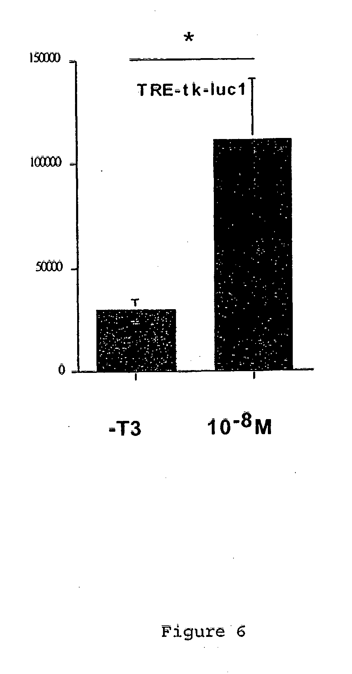

**Figure 6** : Transgenèse somatique par injection dans le muscle dorsal de xénope prétraités de la construction TRE-tk-luciférase. Les animaux sont prétraités 48 heures avec 10⁻¹¹ M d'hormones thyroïdiennes T3, puis rincer pendant 24 heures. Les animaux sont ensuite transfectés par injection dans le muscle dorsal de 1 mg/animal de la construction TRE-tk-luciférase les animaux sont ensuite traités 48 heures avec (10⁻⁸ M) ou sans (-T3) hormone thyroïdienne. 2 jours après le traitement l'expression de la luciférase est mesurée.

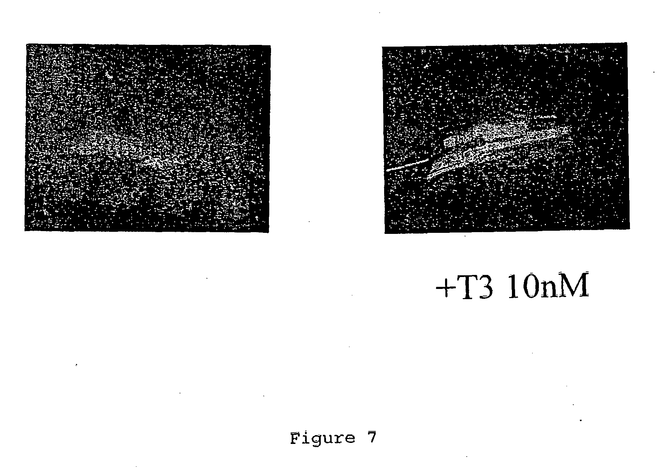

**Figure 7** : Transgenèse somatique par injection dans le muscle dorsal de xénope de la construction TRE-tk-EGFP. Les animaux sont transfectés par injection dans le muscle dorsal de 1mg/animal de la construction TRE-tk-EGFP. Les nimaux sont ensuite traités 48 heures avec (+ T3 10nM) ou sans (-T3) hormone thyroïdienne T3. La bioluminscence est visible dans les cellules de l'animal traité.

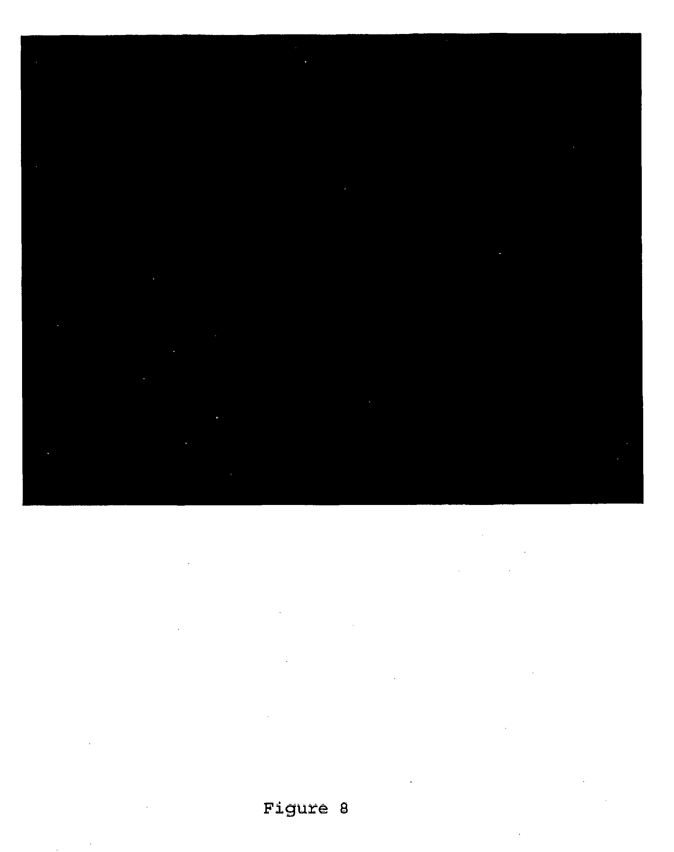

**Figure 8** : Vue ventrale d'un têtard transgénique pour la construction DR4(X3)-GFP avec éléments génétiques insulateurs (« insulators »), répondant à la présence d'hormone thyroïdienne dans l'eau de l'aquarium (10-8M) par l'émission de fluorescence dans l'ensemble du système nerveux et dans les bourgeons de membres.

### EXEMPLES

### 1. Matériels et méthodes

### 1.1. Le vecteur de clonage.

Les cassettes d'expression sont insérées dans le vecteur de base pBluescript II SK (PROMEGA)au niveau du site multiple de clonage contenant notamment les sites de restriction : SacI, SacII, NotI, XbaI, BamHI, SmaI, PstI, EcorI, EcorV, HindIII, SalI, XhoI, ApaI, KpnI.

De préférence, la cassette d'expression est introduite au niveau du site SalI. Au préalable, la cassette et le vecteur ont été linéarisés par SalI et les extrémités ont été rendues franches.

### 1.2. Les éléments de réponse aux récepteurs nucléaires d'hormones.

Différentes séquences de TRE (Thyroid Responsive Element) seront utilisées :
- TRE de l'enzyme malique su rat ( Seq ID N°1)
- TRE consensus en une copie (DR4) (Seq ID N°3, N°5, N°6, N°7, N°9)
- TRE consensus en triple copies (DR4)X3 espacés de 4 nucléotides.

Différentes séquences de séquences promotrices contenant des TRE seront utilisées :
- promoteur de myc murin contenant un TRE négatif (Perez-Juste et al., 2000)(clonage dans les sites PstI-XhoI de pBS-SK II) (Seq ID N°4)
- promoteur du gène de xénope TH/bZIP (Furlow and Brown, 1999) (Seq ID N°12 ; numéro d'accession AF192492 (gi 6707362) genbank) contenant deux TRE positifs (Seq ID N°2 et N°3) (clonage dans les sites SacI-PstI de pBS-SK II) (figure 1).
- Promoteur de la stromélysine 3 de xénope contenant plusieurs TRE(clonage dans le site HindIII).

Les oligonucléotides contenant ces séquences sont clonées dans le vecteur pBS-SK II (PROMEGA) de préférence au niveau des sites XbaI et BamHI.

### 1.3. Le promoteur minimal

Le promoteur minimal de 170 pb du gène de la thymidine kinase (TK) du virus de l'herpès simplex (HSV) est isolé par PCR puis cloné au niveau des sites BamHI-HindIII du pBS-SK II, contenant les séquences de promoteurs contenant les TRE.

Les gènes rapporteurs sont clonés en aval de la séquence TRE-tk.

Ensuite, la séquence « TRE-tk-gène rapporteur » est excisée du vecteur pBS-SK II, puis ses extrémités sont rendues franches afin d'être clonées dans le vecteur contenant les insulateurs.

### 1.4. Le gène rapporteur

Dans un premier temps, les constructions sont réalisées avec les gènes EGFP et luciferase de firefly.

Les constructions suivantes ont ainsi été construites :
- TRE (enzyme malique)-TK-EGFP
- TRE (enzyme malique)-TK-luciférase
- Stromélysine-3-luciférase

Par la suite des gènes de fusion seront générés en associant les séquences codant pour les protéines fluorescentes EGFP ou RedFep (CLONTECH) avec la luciférase de Firefly (PROMEGA) ou de Renilla. Les séquences ADNc de EGFP et RedFep seront amplifiées par PCR en utilisant des amorces contenant des sites de restriction KpnI et BglII. Les fragments amplifiés seront clonés dans ces sites dans le vecteur pGL2 Basic Vector de PROMEGA, qui contient lé gène de la luciferase de firefly. Le bloc de séquence « protéine fluorescente/luciférase/signal de polyadénylation » sera extrait du vecteur pGL2 basic vector par digestion avec les enzymes SmaI et SalI. Les extrémités seront ensuite rendues franches et les fragments de digestion seront clonés en aval des différents promoteurs dans un des sites de restriction du site multiple de clonage du vecteur pBS-SK II qui est libre et situé en 3' des promoteurs, tel par exemple le site SalI.

Les gènes de fusion seront au préalable testés « à vide », c'est-à-dire ils seront clonés dans le site BamHI-HindIII en aval du promoteur TK de HSV dans le pBS-SK II, afin de vérifier l'absence d'éléments cryptiques de réponse aux récepteurs nucléaires d'hormones (notamment TRE) capables d'influencer la réponse des promoteurs et donc de se comporter comme des séquences « enhancers ».

### 1.5. La séquence « insulateur ».

Deux séquences insulateurs sont de préférence utilisées ; celle du gène de la béta-globine de poulet (Seq ID N°11 ; Numéro d'accession AY 040835 (gi : 171 49 284) dans genbank) et celle du gène du lyzozyme de poulet (Seq ID N°10 ; Numéro d'accession X98 408 (gi : 1403311).
Les séquences « insulateurs » sont isolées par réaction de polymérisation en chaine (PCR) en utilisant de l'ADN génomique de poulet ou un vecteur le contenant, comme matrice ADN. Le couple d'amorces utilisées contient des sites de restriction pour faciliter le clonage ultérieur. Ainsi les séquences « insulateurs » du gène de la béta-globine sont clonées dans le vecteur : en position 5' aux niveau des sites SacI-BamHI, en position 3' aux niveau des sites XhoI-KpnI. Les séquences « insulateurs » du gène du lyzozyme sont clonées dans le vecteur : en position 5' aux niveau des sites BamHI-HindIII, en position 3' aux niveau des sites XhoI-KpnI.

Les séquences « insulateurs » ainsi isolées sont clonées de part et d'autre de la cassette d'expression contenant le gène rapporteur sous le controle de séquence régulatrice de la transcription sélectionnée parmi les éléments de réponse aux récepteurs nucléaires d'hormones.

Les séquences définitives « promoteur-gène rapporteur » sont extraits du pBS-SK II et les extrémités sont rendues franches afin d'intégrer ces séquences dans les vecteurs contenant les insulateurs (Figure 2).

### 1.6. Transgenèse somatique

La transgenèse somatique (Ouatas et al., 1998) est utilisée pour vérifier la validité des constructions (Figure 3) et leur potentielle régulation par les composés, tels l'hormone thyroidienne T3, avant l'emploi en transgenèse germinale.

La transgenèse somatique est réalisée soit :
- par injection de 1 µl ADN nu dans le muscle dorsal de l'embryon. L'ADN a une concentration de 100ng/µl à 5 µg/µl dans du NaCL 0,07 M.
- par micro-injection de 1 µl d'ADN/PEI (poléthylèneimine) dans le cerveau de l'embryon. L'ADN a une concentration comprise entre 50 et 500 ng/µl dans du glucose 5%.

La transgenèse germinale est réalisée selon le protocole décrit par Kroll et Amaya (1996).

### 2. Test de fonctionalités des constructions

### 2.1. Tests sur cellules en culture

Les constructions transgéniques sont testées dans les cellules de xénopes XTC en culture.

Les cellules XTC sont obtenues à partir de carcasses de tétards de xénopes juste avant métamorphose (Pudney et *al*., 1973). Les cellules sont cultivées à température ambiante et ce jusqu'à 20 passages.

Les cellules sont transfectées en complexant l'ADN avec du polyéthylène imine (PEI) de 22 kDa à 6 équivalents (Ouatas et *al*., 1998).

Le protocole permet une vérification rapide des constructions et ainsi de vérifier que celles-ci répondent bien aux substances, telles que l'hormone thyroidienne T3 lorsqu'elle est injectée dans le milieu de culture des cellules.

Des cellules XTC seront produites à partir de tétards transgéniques afin de réaliser des tests. Ces cellules XTC transgéniques pourront être transfectées par des vecteurs surexprimant différentes isoformes de récepteurs nucléaires des hormones pour étudier l'action des perturbateurs endocriniens.

### 2.2. Transfection de cellule XTC avec la construction stromélysine 3/ luciférase.

En absence d'hormone thyroidienne T3 dans le milieu de culture des cellules XTC transgéniques, un niveau d'expression basale de la protéine rapporteuse luciférase est observée. L'adjonction d'hormone thyroïdienne T3 au milieu de culture à une concentration de 10⁻⁸ M induit l'expression plus importante de la protéine rapporteuse (Figure 4).

### 3. Transgenèse somatique chez le xénope avec TRE-TK-Luciférase

3.1. La construction TRE-tk-luciférase a été injectée dans le muscle dorsal d'embryon de xénope (1 mg/animal). La moitié des animaux sont traités avec de l'hormone thyroidienne T3 à 10⁻⁸ M. Quatre jours après, l'expression du gène rapporteur est mesurée chez les animaux traités (10⁻⁸ M de T3) et non traités (- T3). L'adjonction d'hormone thyroidienne T3 au milieu de culture à une concentration de 10⁻⁸ M induit l'expression beaucoup plus importante de la protéine rapporteuse (Figure 5).
3.2. Selon une mode alternatif, les animaux transgéniques selon l'invention sont au préalable traités au perchlorate pendant 1 mois à 1g/l puis sont sensibilisés par le composé à tester, telles l'hormone T3 (10⁻¹¹ M) pendant 18 à 48 heures sans nourriture. Les animaux sont ensuite lavés dans un milieu ne contenant pas de T3 et nourris pendant 36 heures. La construction (par exemple TRE-tk-luciférase) est injectée dans le muscle dorsal de l'embryon de xénope (1 mg/animal).
   Sur les 24 tétards prétraités, puis soumis à la transgenèse somatique, douze tétards sont traités pendant 48 heures avec de l'hormone thyroidienne T3 à 10⁻⁸ M ; les douze autres tétards restant servant de controle. L'adjonction d'hormone thyroidienne T3 au milieu de culture à une concentration de 10⁻⁸ M induit l'expression beaucoup plus importante de la protéine rapporteuse (Figure 6).
3.3. La construction TRE-tk-EGFP a été injectée dans le muscle dorsal d'embryon de xénope (1 mg/animal). La moitié des animaux sont traités avec de l'hormone thyroidienne T3 à 10 nM. Quatre jours après, l'expression du gène rapporteur est détectée chez les animaux traités (10 nM de T3) et non traités (- T3). L'adjonction d'hormone thyroidienne T3 au milieu de culture à une concentration de 10 nM induit l'expression beaucoup plus importante de la protéine rapporteuse (Figure 7).

### REFERENCES

Arnheiter et al., (1990) Cell 62 : 51
Argenton et al. (1996) Mol Cell Biol 16 : 1714-1724
Brinster et al., (1982) Nature 296 : 39-42
Brown et al., (1987) Cell 49 : 603-612
Brown et al., (1996) Proc. Natl. Acad. Sci. USA 93: 1924-9
Capecchi et al., (1989) Science 244 :1288-1291
Ciana et al. (2001) Mol. Endocrinol 15 : 1104-13
Coen et al. (2001) Proc. Natl. Acad. Sci. USA 98 : 7869-74
Day et al., (1998) Biotechniques 25: 848-50, 852-4, 856
De Luze et al., (1993) Proc. Natl. Acad. Sci. USA 90: 7322-6
Denver et al., (1997) J. Biol. Chem 272: 8179-88
Denver et al., (1999) J. Biol. Chem 274 (33) : 23128-34
Deuschle et al., (1990) Science 2 : 480-483
Echt et al. (1988) Embo J. 7 : 2063-2073
Farsetti et al. (1992) J. Biol. Chem 267: 15784-8
Figge et al., (1988) Cell 49 : 603-612
Furlow et al. (1999) Mol. Endocrinol 13: 2076-89
Gossen et al., (1992) Proc. Natl. Acad. Sci. USA 89: 5547-5551)
Hu et al., (1987) Cell 48 : 555-556
Hug et al., (1998) Mol. Cell. Biol. 8:3065
Hynes et al., (1981) Proc. Natl. Acad. Sci. USA 78 : 2038-2042
Ishizuya-Oka et al. (1996) Cell Tissue Research
Israel et al., (1989) Nucleic Acids Res. 17 : 2589-2604
Karsenti et al. (2000) Semin. Cell. Biol 11(5) : 343-6
Kitazawa et al. (2002) Biochel Biophys Res Commun 290: 650-655
Klock et al., (1987) Nature 329: 734-736
Kolla et al. (1998) Biophys Res Commun. 266: 5 -14
Kroll et al., (1996) Development 122: 3173-83
Kroll et Amaya , (1999) Methods Mol Biol 97: 393-414
Labow et al., (1990) Mol. Cell. Biol. 10: 3343-3356)
Lee et al., (1981) Nature 294 : 228-232
Liu et al., (2000) Luminescence 15: 45-9
Luo et al., (2001) Biochem Biophys Res Commun
Mader et al. (1993) Nucleic Acids Res 21: 1125-1132
Mayo et al., (1982) Cell 29 : 99-108
Metzger et Feil (1999), Curr. Opin. Biotechnol. 5 : 470-476
Namciu et al. (1998) Mol. Cell. Biol 18: 2382-91
Nover et al., (1991) in « Heat Shock Response », e.d.
Nover, L CRC, Boca Raton, Fl pp. 167-220
Oofusa et al.,(2001) Mol. Cell. Endocrinol 181: 97-110
Ouatas et al., 1998) Int. J. Dev. Biol 42: 1159-64
Perez-Juste et al.,(2000) J. Biol. Chem 275: 1307-14
Pudney et al. (1973) Experiantia 29: 466-467
Roberto et al., (2000) Endocrinology 141: 4056-4064
Schmidt et al., (1990) Mol. Cell. Biol. 10 : 4406-4411
Seark et al., 1985 Mol. Cell. Biol. 5: 1480-1489
Stief et al., (1989) Nature 341: 343-5
Wang et al., (1993) J. Biol. Chem 268 (22): 16270-8
Xiang et al., (1998) Nucleic Acids Research 26: 2034-2035

## Revendications

1. Animal aquatique transgénique obtenu par transgénèse germinale sélectionné parmi les amphibiens et les téléostes comprenant au moins une cassette d'expression, la dite cassette comprenant :
a) une séquence d'ADN régulatrice de vertébré comprenant le promoteur TH/bZIP de séquence SEQ ID No 12 lié de manière opérationnelle en amont d'un segment d'ADN codant pour une protéine rapporteuse,
b) facultativement un signal de polyadénylation, et
c) une séquence "insulateur" à chaque extrémité de la dite cassette.

2. Animal selon la revendication 1 **caractérisé en ce que** la dite protéine rapporteuse est sélectionnée dans le groupe des protéines auto-fluorescentes et des enzymes détectables par un procédé histo-chimique.

3. Animal selon la revendication 2 **caractérisé en ce que** la dite protéine fluorescente est choisie dans le groupe composé de la protéine de fluorescence verte (GFP), la protéine de fluorescence verte augmentée (EGFP), la protéine de fluorescence rouge (CFP), la protéine de fluorescence bleue (BFP), la protéine de fluorescence jaune (YFP), et les variants fluorescents de ces protéines, les protéines fluorescentes changeant de couleur avec le temps ( « fluorescent timer »), les protéines fluorescentes dont la fluorochrome a une durée de vie très courte.

4. Animal selon la revendication 2 **caractérisé en ce que** la dite enzyme détectable par un procédé histochimique est choisie dans le groupe composé de la luciférase, la β-galactosidase, la β-glucuronidase, la phosphatase alcaline, la chloramphénicol-acétyltransférase, la déshydrogénase alcoolique.

5. Animal selon les revendications 1 à 4 **caractérisée en ce que** la dite cassette d'expression est insérée de manière stable dans le génome cellulaire.

6. Animal selon les revendications 1 à 5 **caractérisé en ce que** le dit amphibien est la grenouille sélectionnée de préférence parmi *Xénopus laevis* et *Xenopus tropicalis,* et le dit téléoste est sélectionné parmi le poisson zèbre (zebrafish) et medaka.

7. Grenouille transgénique et ses descendants selon la revendication 6, aux différents stades de leur développement, et de préférence au stade têtard, obtenue par transgénèse germinale, **caractérisée en ce que** l'ensemble des cellules de l'animal sont transgéniques.

8. Grenouille transgénique selon la revendication 7 au stade têtard.

9. Procédé pour identifier la présence d'au moins un polluant environnemental qui module, de préférence stimule, la transcription médiée par des éléments de réponse aux récepteurs nucléaires d'hormones, comprenant les étapes de :
a) mise en contact d'un animal selon l'une des revendications 1 à 8 dans un milieu aqueux comprenant le polluant environnemental ;
b) mise en contact d'un animal selon l'une des revendications 1 à 8 dans un milieu aqueux ;
c) détermination qualitative, facultativement quantitative, de l'expression de la protéine rapporteuse en a) et b) puis comparaison desdites expressions ;
de sorte qu'une différence d'expression de la dite protéine rapporteuse en a) et b) indique la présence de polluants environnementaux dans le milieu.

10. Procédé de criblage de composé (s) modulant, de préférence stimulant, la transcription médiée par des éléments de réponse aux récepteurs nucléaires d'hormones, comprenant les étapes de :
a) mise en contact d'un animal selon l'une des revendications 1 à 8 dans un milieu aqueux comprenant le ou les composés ;
b) mise en contact d'un animal selon l'une des revendications 1 à 8 dans un milieu aqueux ;
c) détermination qualitative, facultativement quantitative de l'expression de la protéine rapporteuse en a) et b) puis comparaison desdites expressions ;
d) sélection du ou des composés induisant une différence d'expression de la dite protéine rapporteuse en a) et b).

11. Utilisation du procédé selon la revendication 10 pour l'analyse et l'étude du mode d'action biologique de composé (s) modulant la transcription médiée par des éléments de réponse aux récepteurs nucléaires d'hormones.

12. Utilisation du procédé selon la revendication 9 pour étudier l'effet de dose des polluants environnementaux qui stimulent la transcription médiée par des éléments de réponse aux récepteurs nucléaires d'hormones.

13. Utilisation d'un animal selon l'une des revendications 1 à 8, pour la détection, l'analyse et/ou l'étude des contaminants environnementaux, notamment dans l'eau.

14. Utilisation d'un animal selon l'une des revendications 1 à 8 pour analyser et/ou évaluer la qualité de l'eau.

15. Utilisation d'un animal selon l'une des revendications 1 à 8 pour la réalisation d'étude de toxicité et/ou de toxicologie, notamment en cosmétologie, en agrochimie, en pharmacie.

16. Utilisation d'un animal selon l'une des revendications 1 à 8 dans un protocole d'étude de la tératogénèse sur des embryons de grenouille (FETAX pour Frog Embryo Teratogenicity assay).

17. Dispositif pour la détection de polluants environnementaux, notamment dans l'eau, et/ou de composé (s), modulant la transcription médiée par des éléments de réponse aux récepteurs nucléaires d'hormones **caractérisé en ce qu'**il comprend un animal selon l'une des revendications 1 à 8, et facultativement des moyens de détection de l'expression de ou des gènes rapporteur.

## Patentansprüche

1. Transgenes Wassertier, erhalten durch Keimbahn-Transgenese, ausgewählt aus den Amphibien und den Echten Knochenfischen, mindestens eine Expressionskassette umfassend, wobei die Kassette umfasst:
a) eine regulierende Wirbeltier-DNA-Sequenz, die den Promoter TH/bZIP der Sequenz SEQ ID Nr. 12 umfasst, der operativ vor einem für ein Reporterprotein kodierenden DNA-Segment gebunden ist,
b) fakultativ ein Polyadenylierungssignal und
c) eine Isolatorsequenz an jedem Ende der Kassette.

2. Tier nach Anspruch 1, **dadurch gekennzeichnet, dass** das Reporterprotein aus der Gruppe der selbstfluoreszierenden Proteine und der durch ein histochemisches Verfahren nachweisbaren Enzyme ausgewählt ist.

3. Tier nach Anspruch 2, **dadurch gekennzeichnet, dass** das fluoreszierende Protein aus der Gruppe ausgewählt ist, die sich aus dem grün fluoreszierenden Protein (GFP), dem verstärkt grün fluoreszierenden Protein (EGFP), dem rot fluoreszierenden Protein (CFP), dem blau fluoreszierenden Protein (BFP), dem gelb fluoreszierenden Protein (YFP) und den fluoreszierenden Varianten dieser Proteine zusammensetzt, wobei die Proteine mit der Zeit die Farbe wechseln (Fluoreszenz-Timer), wobei die fluoreszierenden Proteine, darunter das Fluorochrom, eine sehr kurze Lebensdauer haben.

4. Tier nach Anspruch 2, **dadurch gekennzeichnet, dass** das durch ein histochemisches Verfahren nachweisbare Enzym aus der Gruppe ausgewählt ist, die aus der Luciferase, der β-Galactosidase, der β-Glucuronidase, der alkalischen Phosphatase, der Chloramphenicolacetyltransferase, der alkoholischen Dehydrogenase zusammengesetzt ist.

5. Tier nach den Ansprüchen 1 bis 4, **dadurch gekennzeichnet, dass** die Expressionskassette stabil in das zelluläre Genom eingesetzt ist.

6. Tier nach den Ansprüchen 1 bis 5, **dadurch gekennzeichnet, dass** die Amphibie der Frosch ist, der vorzugsweise aus *Xenopus laevis* und *Xenopus tropicalis* ausgewählt ist, und dass der Echte Knochenfisch aus dem Zebrafisch und dem Medaka ausgewählt ist.

7. Transgener Frosch und seine Nachkommen nach Anspruch 6 in verschiedenen Stadien ihrer Entwicklung und vorzugsweise im Stadium Kaulquappe, erhalten durch Keimbahn-Transgenese, **dadurch gekennzeichnet, dass** alle Zellen des Tiers transgen sind.

8. Transgener Frosch nach Anspruch 7 im Stadium Kaulquappe.

9. Verfahren zur Identifizierung der Anwesenheit mindestens eines Umweltschadstoffs, der die von Response Elementen an hormonalen Kernrezeptoren vermittelte Transkription moduliert, vorzugsweise stimuliert, das die folgenden Schritte umfasst:
a) Inkontaktversetzen eines Tiers nach einem der Ansprüche 1 bis 8 in einem wässrigen Milieu, das den Umweltschadstoff umfasst;
b) Inkontaktversetzen eines Tiers nach einem der Ansprüche 1 bis 8 in einem wässrigen Milieu;
c) qualitatives, fakultativ quantitatives Bestimmen der Expression des Reporterproteins in a) und b), danach Vergleichen der Expressionen,
so dass eine Expressionsdifferenz des Reporterproteins in a) und b) die Anwesenheit von Umweltschadstoffen in dem Milieu anzeigt.

10. Screening-Verfahren von Verbindung(en), das die von Response Elementen an hormonalen Kernrezeptoren vermittelte Transkription moduliert, vorzugsweise stimuliert, das die folgenden Schritte umfasst:
a) Inkontaktversetzen eines Tiers nach einem der Ansprüche 1 bis 8 in einem wässrigen Milieu, das die Verbindung(en) umfasst;
b) Inkontaktversetzen eines Tiers nach einem der Ansprüche 1 bis 8 in einem wässrigen Milieu;
c) qualitatives, fakultativ quantitatives Bestimmen der Expression des Reporterproteins in a) und b), danach Vergleichen der Expressionen;
d) Auswahl der Verbindung(en), die eine Expressionsdifferenz des Reporterproteins in a) und b) induziert/induzieren.

11. Verwendung des Verfahrens nach Anspruch 10 für die Analyse und die Untersuchung des biologischen Aktionsmodus von Verbindung(en), die die von Response Elementen an hormonalen Kernrezeptoren vermittelte Transkription modulieren.

12. Verwendung des Verfahrens nach Anspruch 9 für die Untersuchung des Dosiseffekts der Umweltschadstoffe, die die von Response Elementen an hormonalen Kernrezeptoren vermittelte Transkription modulieren.

13. Verwendung eines Tiers nach einem der Ansprüche 1 bis 8 für den Nachweis, die Analyse und/oder die Untersuchung von Umweltkontaminanten, insbesondere im Wasser.

14. Verwendung eines Tiers nach einem der Ansprüche 1 bis 8 zum Analysieren und/oder Beurteilen der Wasserqualität.

15. Verwendung eines Tiers nach einem der Ansprüche 1 bis 8 für die Durchführung von Toxizitäts- und/oder toxikologischen Untersuchungen, insbesondere in der Kosmetologie, in der Agrochemie, in der Pharmazie.

16. Verwendung eines Tiers nach einem der Ansprüche 1 bis 8 in einem Studienprotokoll der Teratogenese an Froschembryonen (FETAX für Frog Embryo Teratogenicity Assay).

17. Vorrichtung für den Nachweis von Umweltschadstoffen, insbesondere im Wasser, und/oder von Verbindung(en), die die von Response Elementen an hormonalen Kernrezeptoren vermittelte Transkription moduliert/modulieren, **dadurch gekennzeichnet, dass** sie ein Tier nach einem der Ansprüche 1 bis 8 und fakultativ Nachweismittel der Expression von Reportergen(en) umfasst.

## Claims

1. A transgenic aquatic animal obtained by germinal transgenesis selected from amphibians and teleosts, comprising at least one expression cassette, said cassette comprising:
a) a regulatory DNA sequence from vertebrate, comprising the promoter TH/bZIP of sequence SEQ ID NO:12 functionally linked upstream of a DNA segment encoding a reporter protein,
b) optionally, a polyadenylation signal, and
c) an "insulator" sequence at each end of said cassette.

2. The animal according to claims 1, **characterized in that** said reporter protein is selected in the group of autofluorescent proteins and enzymes detectable by a histochemical method.

3. The animal according to claim 2, **characterized in that** said fluorescent protein is chosen in the group composed of the green fluorescent protein (GFP), the enhanced green fluorescent protein (EGFP), the red fluorescent protein (CFP), the blue fluorescent protein (BFP), the yellow fluorescent protein (YFP), and the fluorescent variants of these proteins, the fluorescent proteins that change color with time (fluorescent timer), and the fluorescent proteins for which the fluorochrome has a very short lifetime.

4. The animal according to claim 2, **characterized in that** said enzyme detectable by a histochemical method is chosen in the group composed of luciferase, β-galactosidase, β-glucuroniclase, alkaline phosphatase, chloramphenicol acetyl transferase and alcohol dehydrogenase.

5. The animal according to claims 1 to 4, **characterized in that** said expression cassette is inserted stably into the cell genome.

6. The animal according to claims 1 to 5, **characterized in that** said amphibian is the frog, preferably selected among *Xenopus laevis* and *Xenopus tropicalis,* and said teleost is selected among the zebrafish and medaka.

7. A transgenic frog and its progeny according to claim 6, at the various stages of their development, and preferably at the tadpole stage, obtained by germinal transgenesis, **characterized in that** all the cells of the animal are transgenic.

8. The transgenic frog according to claim 7, at the tadpole stage.

9. A method for identifying the presence of at least one environmental pollutant which modulates, preferably stimulates, transcription mediated by nuclear hormone receptor response elements, comprising the steps of:
a) contacting an animal according to one of claims 1 to 8, in an aqueous medium comprising the environmental pollutant;
b) contacting an animal according to one of claims 1 to 8, in an aqueous medium;
c) qualitatively, optionally quantitatively, determining the expression of the reporter protein in a) and b) and then comparing said expressions;
such that a difference in expression of said reporter protein in a) and b) indicates the presence of environmental pollutants in the medium.

10. A method for screening (a) compound(s) which modulate(s), preferably stimulate(s), transcription mediated by nuclear hormone receptor response elements, comprising the steps of:
a) contacting an animal according to one of claims 1 to 8, in an aqueous medium comprising the compound(s);
b) contacting an animal according to one of claims 1 to 8, in an aqueous medium;
c) qualitatively, optionally quantitatively, determining the expression of the reporter protein in a) and b), and then comparing said expressions;
d) selecting the compound(s) that induce(s) a difference in expression of said reporter protein in a) and b).

11. Use of the method according to claim 10, for analyzing and studying the mode of biological action of (a) compound(s) which modulate(s) transcription mediated by nuclear hormone receptor response elements.

12. The use of the method according to claim 9, for studying the dose effect of environmental pollutants which stimulate transcription mediated by nuclear hormone receptor response elements.

13. The use of an animal according to one of claims 1 to 8, for detecting, analyzing and/or studying environmental contaminants, in particular in water.

14. The use of an animal according to one of claims 1 to 8, for analyzing and/or evaluating the quality of water.

15. The use of an animal according to one of claims 1 to 8, for carrying out a study of toxicity and/or toxicology, in particular in cosmetology, in agrochemistry or in pharmacy.

16. The use of an animal according to one of claims 1 to 8, in a protocol for studying teratogenesis on frog embryos (FETAX for Frog Embryo Teratogenicity assay).

17. A device for detecting environmental pollutants, in particular in water, and/or (a) compound(s), which modulate transcription mediated by nuclear hormone receptor response elements, **characterized in that** it comprises an animal according to one of claims 1 to 8, and, optionally, means for detecting the expression of the reporter gene(s).


### LISTE DE SEQUENCES

<110> CNRS Centre National de la Recherché Scientifique
<120> Embryons transgéniqués de xénope et leurs utilisations pour la détection de perturbateurs endocriniens et procédés correspondants
<130> D19860
<150> FR 02/06 669
   <151> 2002-05-30
<160> 12
<170> PatenIn Ver. 2.1
<210> 1
   <211> 20
   <212> ADN
   <213> Rattus sp.
<400> 1
   ttggggttag gggaggacag 20
<210> 2
   <211> 20
   <212> ADN
   <213> Xenopus sp.
<400> 2
   ctagggttaa gtaaggtgaa 20
<210> 3
   <211> 20
   <212> ADN
   <213> Xenopus sp.
<400> 3
   ctaaggtaag cccgggttag 20
<210> 4
   <211> 19
   <212> ADN
   <213> Xenopus sp.
<400s 4
   gggcgaccta agaaggcag 19
<210> 5
   <211> 20
   <212> ADN
   <213> Xenopus sp.
<400> 5
   ggcaggtcat ttcaggacag 20
<210> 6
   <211> 19
   <212> ADN
   <213> Xenopus sp.
<400> 6
   gtctgacact geegacctc 19
<210> 7
   <211> 16
   <212> ADN
   <213> Xenopus sp.
<400> 7
   aggtcagtta aggtga 16
<210> 8
   <211> 14
   <212> ADN
   <213> Xenopus sp.
<400> 8
   aggtgaacag gaca 14
<210> 9
   <211> 16
   <212> ADN
   <213> Xenopus sp.
<400> 9
   gggtacatat aggtca 16
<210> 10
   <211> 1668
   <212> ADN
   <213> Gallus gallus
<400> 10
   ggatccataa tataactgta ccaggttttg gtttattaca tgtgactgac ggcttcctat 60
   gcgtgctcag aaaacggcag ttgggcactg cactgcccgg tgatggtgcc acggtggctc 120
   ctgccgcctt ctttgatatt cactctgttg tatttcatct cttgttgccg atgaaaggat 180
   ataacagtct ctgaggaaat acttggtatt tcttctgatc agcgttttta taagtaatgt 240
   tgaatattgg ataaggctgt gtgtcctttg tcttgggaga caaagcccac agcaggtggt 300
   ggttgggtgg tggcagctca gtgacaggag aggttttttt gcctgttttt tttgttgttt 360
   ttttttttta agtaaggtgt tcttttttct tagtaaaatt tctactggac tgtatgtttt 420
   gacaggtcag aaacatttct tcaaaagaag aaccttttgg aaactgtaca gcccttttct 480
   ttcattccct ttttgctttc tgtgccaatg cctttggttc tgattgcatt atggaaaacg 540
   ttgatcggaa cttgaggttt ttatttatag tgtggcttga aagcttggat agctgttgtt 600
   acatgagata ccttattaag tttaggccag cttgatgctt tatttttttt cctttgaagt 660
   agtgagcgtt ctctggtttt tttcctttga aactggcgag gcttagattt ttctaatggg 720
   attttttacc tgatgatcta gttgcatacc caaatgcttg taaatgtttt cctagttaac 780
   atgttgataa cttcggattt acatgttgta tatacttgtc atctgtgttt ctagtaaaaa 840
   tatatggcat ttatagaaat acgtaattcc tgatttcctt ttttttttat ctctatgctc 900
   tgtgtgtaca ggtcaaacag acttcactcc tatttttatt tatagaattt tatatgcagt 960
   ctgtcgttgg ttcttgtgtt gtaaggatac agccttaaat ttcctagagc gatgctcagt 1020
   aaggcgggtt gtcacatggg ttcaaatgta aaacgggcac gtttgctgct gccttcccag 1080
   atccaggaca ctaaactgct tctgcaactg aggtataaat cgcttcagat cccaggaagt 1140
   gtagatccac gtgcatattc ttaaagaaga atgaatactt tctaaaatat gttggcatag 1200
   gaagcaagct gcatggattt atttgggact taaattattt tggtaacgga gtgcataggt 1260
   tttaaacaca gttgcagcat gctaacgagt cacagcattt atgcagaagt gatgcctgtt 1320
   gcagctgttt acggcactgc cttgcagtga gcattgcaga taggggtggg gtgctttgtg 1380
   tcgtgttggg acacgctgcc acacagccac ctcccgaaca tatctcacct gctgggtact 1440
   tttcaaacca tcttagcagt agtagatgag ttactatgaa acagagaagt tcctcagttg 1500
   gatattctca tgggatgtct tttttcccat gttgggcaaa gtatgataaa gcatctctat 1560
   ttgtaaatta tgcacttgtt agttcctgaa tcctttctat agcaccactt attgcagcag 1620
   gtgtaggctc tggtgtggcc tgtgtctgtg cttcaatctt ttaagctt 1668
<210> 11
   <211> 1216
   <212> ADN
   <213> Gallus gallus
<400> 11
   tctagaggga cagccccccc ccaaagcccc cagggatgta attacgtccc tcccccgcta 60
   gggggcagca gcgagccgcc cggggctccg ctccggtccg gcgctccccc cgcatccccg 120
   agccggcagc gtgcggggac agcccgggca cggggaaggt ggcacgggat cgctttcctc 180
   tgaacgcttc tcgctgctct ttgagcctgc agacacctgg ggggatacgg ggaaaaagct 240
   ttaggctgaa agagagattt agaatgacag aatcatagaa cggcctgggt tgcaaaggag 300
   cacagtgctc atccagatcc aaccccctgc tatgtgcagg gtcatcaacc agcagcccag 360
   gctgcccaga gccacatcca gcctggcctt gaatgcctgc agggatgggg catccacagc 420
   ctccttgggc aacctgttca gtgcgtcacc accctctggg ggaaaaactg cctcctcata 480
   tccaacccaa acctcccctg tctcagtgta aagccattcc cccttgtcct atcaaggggg 540
   agtttgctgt gacattgttg gtctggggtg acacatgttt gccaattcag tgcatcacgg 600
   agaggcagat cttggggata aggaagtgca ggacagcatg gacgtgggac atgcaggtgt 660
   tgagggctct gggacactct ccaagtcaca gcgttcagaa cagccttaag gataagaaga 720
   taggatagaa ggacaaagag caagttaaaa cccagcatgg agaggagcac aaaaaggcca 780
   cagacactgc tggtccctgt gtctgagcct gcatgtttga tggtgtctgg atgcaagcag 840
   aaggggtgga agagcttgcc tggagagata cagctgggtc agtaggactg ggacaggcag 900
   ctggagaatt gccatgtaga tgttcataca atcgtcaaat catgaaggct ggaaaagccc 960
   tccaagatcc ccaagaccaa ccccaaccca cccaccgtgc ccactggcca tgtccctcag 1020
   tgccacatcc ccacagttct tcatcacctc cagggacggt gaccccccca cctccgtggg 1080
   cagctgtgcc actgcagcac cgctctttgg agaaggtaaa tcttgctaaa tccagcccga 1140
   ccctcccctg gcacaacgta aggccattat ctctcatcca actccaggac ggagtcagtg 1200
   aggatggggc tctaga 1216
<210> 12
   <211> 836
   <212> ADN
   <213> Xenopus lâévis
<400> 12
   gatctccctg ctctttataa aatgactgaa tgtctccctg ttgtgtataa tggtgcagag 60
   tatgttatgg ttgctaatag actgagctgt tatatagagg cagagggaca tgttgatagg 120
   ctgtgcattc agtacacatg agtagcgagt gccgttcagg agttgagaga ggggagaggg 180
   aggcttgggg agttgtgagc aagagaaggg gacagagtga gtgagaggag gggaaaggct 240
   ggcacatttt cacaggacgc actagggtta agtaaggtga atgctcagcc tcatttgaac 300
   tctgtagggt tgggagagac acacacgctg tgtatataca tagtgataga gagagggggc 360
   cgggagcgag acaggggctg gggagagttc agcacaggag agagaggcag ggagagggac 420
   aaagagacac ttagcaacag ggatcagctt ggggacaagt gagaagcaag agcaagttct 480
   gctgcccatt caggtatagt ataacctgca atctgcatgc aactgccatg tactcccact 540
   cccacctctc ttctactgca tatacactga tagcacagcc tagtctgctg agcttacact 600
   ctaatataca gtgagacaca gatacaatac agggagagga actattggga gaagagaata 660
   ctgctctgca aagcttacac tccattatac agagggacac agatacaata cagagaggga 720
   accattggta gaagagagta ctgccctgca gagcttacac tctagtgtac aggcggacac 780
   ataaacactt catggaaatg ggagcaaatt aggtaaagaa tagatttcag aaagat 836